# EUROPEAN PATENT SPECIFICATION

(11) **EP 2 870 924 B1**
(45) Date of publication and mention of the grant of the patent: **30.12.2020**
(21) Application number: 14192145.2
(22) Date of filing: 06.11.2014
(51) Int. Cl.: A61B 17/064, A61B 17/068

(54) **Surgical fastener**
Chirurgisches Befestigungselement
Dispositif de fixation chirurgical

(30) Priority: 08.11.2013 US 201314075464
(43) Date of publication of application: 13.05.2015
(73) Proprietor: C.R. BARD, INC., Murray Hill New Jersey 07974 (US)
(72) Inventor: Ranucci, Kevin J., Warwick, Rhode Island 02886 (US); Gupta, Saurav V., Medway, Massachusetts 02053 (US)
(74) Representative: Grey, Ian Michael

(56) References cited:
- WO-A1-2005/004727
- WO-A2-2012/176195
- US-A1- 2007 038 220
- US-A1- 2010 256 690
- US-A1- 2012 101 526

## Description

Disclosed embodiments are related to a surgical fastener, and more particularly, to a surgical fastener that includes a coil body with an attached head.

Surgical fasteners are widely used in many different medical procedures. For example, staples, sutures, clips and other fasteners are commonly used in laparoscopic and open surgical procedures.

It is known from WO2005/004727 A1, to provide a head including a head body and at least two radial wings extending in an outward radial direction from the head body, each wing including at least one external thread to engage with a corresponding internal thread of a delivery device, each wing being spaced from an adjacent wing by an opening therebetween that is adapted to receive a drive member for driving the surgical fastener; and a coil body that is separate from, and is attached to, the head, the coil body including a plurality of coil windings, the coil body having a proximal end and a distal end, the proximal end being attached to a centre portion of the head.

A surgical fastener according to the present invention is characterised in that the head and the coil body are configured so that a gap is formed between the proximal-most coil winding of the coil body and the head; the coil body includes a central post at the proximal end that is attached to the center portion of the head and includes an axial portion and a radial portion that extends from a proximal end of the axial portion, wherein the radial portion is configured for enhanced holding between the head and the coil body.

Preferred features of the surgical fastener of the invention are defined in the dependent claims numbered 2 to 11.

Various embodiments of the invention will now be described, by way of example, with reference to the accompanying drawings, in which:
FIG. 1 is a schematic front view of a surgical fastener;
FIG. 2 is a schematic side view of the surgical fastener of FIG. 1;
FIG. 3 is a schematic top view of the surgical fastener of FIG. 1;
FIG. 4 is a schematic perspective view of the surgical fastener of FIG. 1;
FIG. 5 is a schematic perspective view of the surgical fastener of FIG. 1;
FIG. 6 is a schematic front view of a surgical fastener head;
FIG. 7 is a schematic perspective view of the surgical fastener head of FIG. 6;
FIG. 8 is a schematic top view of the surgical fastener head of FIG. 6;
FIG. 9 is a schematic side view of the surgical fastener head of FIG. 6;
FIG. 10 is a schematic cross-sectional view of the surgical fastener head of FIG. 6;
FIG. 11 is a schematic top view of a surgical fastener coil body;
FIG. 12 is a schematic perspective view of the surgical fastener coil body of FIG. 11;
FIG. 13 is a schematic front view of the surgical fastener coil body of FIG. 11;
FIG. 14 is a schematic side view of the surgical fastener coil body of FIG. 11; and
FIG. 15 is a schematic cross-sectional view of a delivery device.

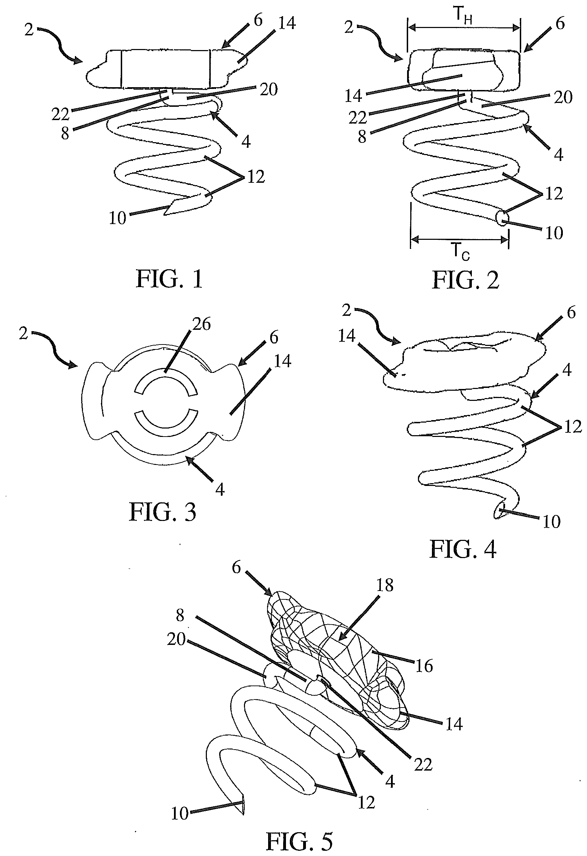

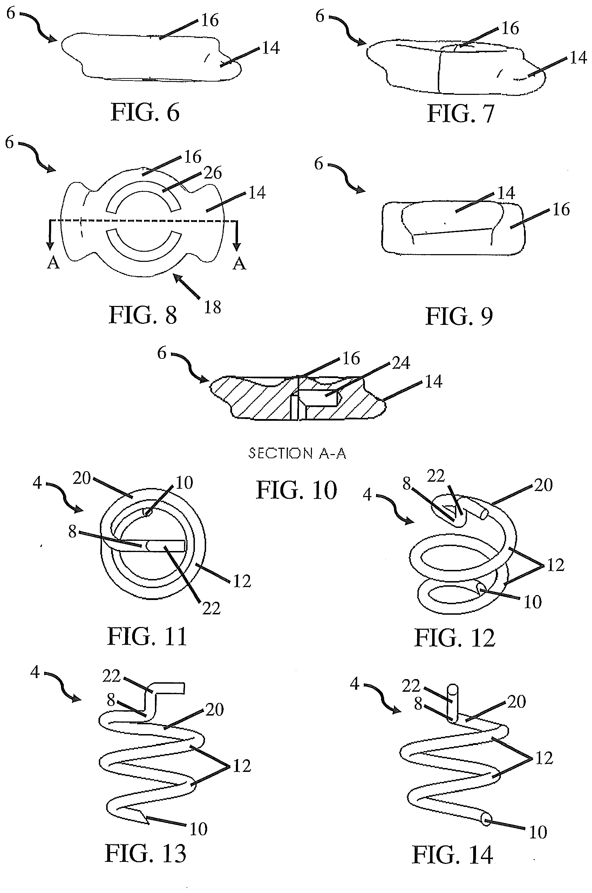

Examples are described with reference to Figures 1 to 9, and embodiments of the invention are defined with reference to Figures 10 to 15.

It should be understood that aspects of the invention are described herein with reference to the figures, which show illustrative embodiments in accordance with aspects of the invention. The illustrative embodiments described herein are not necessarily intended to show all aspects of the invention, but rather are used to describe a few illustrative embodiments. Thus, aspects of the invention are not intended to be construed narrowly in view of the illustrative embodiments. In addition, it should be understood that aspects of the invention may be used alone or in any suitable combination with other aspects of the invention.

A surgical fastener is provided for various surgical fastening applications. For example, the surgical fastener may be used to attach an implantable prosthesis, such as a soft tissue repair fabric, to tissue and/or muscle. Other non-limiting applications for the fastener may involve joining portions of tissue and/or muscle together, joining portions of tissue and/or muscle to bone, and/or joining an implantable prosthesis to bone.

The surgical fastener includes a coil body and a separate head that is attached to the coil body. This arrangement may improve the manufacturability of the fastener and reduce costs, particularly as compared to costs associated with injection molding a complex surgical fastener. This arrangement may be particularly suited for manufacturing the head and coil body from different materials.

For the purposes of this application, a transverse dimension of the coil body or head generally refers to a dimension of the coil body or head within a plane that is perpendicular to a long axis of the surgical fastener when it is assembled (e.g. a diameter of a cylindrical coil body, a width of a rectangular head, the length of a side of a triangular coil body, etc...). For example, an outer transverse dimension of the coil body would refer to the lateral distance between opposing outer surfaces of the coil body and an inner transverse dimension of the coil body would refer to the lateral distance between opposing interior surfaces of the coil body. The outer transverse dimensions of the head T_{H} and coil body Tc in one embodiment are illustrated in Fig. 2 and correspond to the width of the head and the diameter of the coil body. It should be noted that in embodiments in which the head and/or the coil body are noncircular, the head and/or coil body may have both minimum and maximum transverse dimensions.

In one embodiment, the head may be configured to be larger, such as wider or greater in diameter, than a transverse dimension of the coil body to engage and secure underlying material and/or tissue. Additionally, the head may include one or more features that cooperate with corresponding features of a delivery device for driving the fastener from the device and into an implantable prosthesis and/or tissue or muscle.

The head includes two or more radial wings extending in an outward radial direction from a head body. Each wing includes an external thread to engage with a corresponding internal thread of a delivery device. Each wing is also spaced from an adjacent wing by an opening, such as a slot, positioned therebetween and adapted to receive a drive member for driving the surgical fastener. The coil body may have a smaller maximum outer transverse dimension than the wings so that the coil windings do not interact with the internal thread of the delivery device when the external threads of the wings engage the internal thread. The coil body may also have a smaller maximum outer transverse dimension than the head body so that the coils do not interfere with the drive member received within the slots. The head may also include one or more openings for supporting tissue ingrowth thereto.

The coil body is attached to the center of the head. A proximal-most coil winding of the coil body is spaced from a distal face of the head to form a gap therebetween. The coil body includes a central post at the proximal end that is attached to the center of the head. The central post is shaped to facilitate retention of the head and has an axial portion and a radial portion that extends from a proximal end of the axial portion to aid in attaching and retaining the central post relative to the head.

In the example shown in FIGS. 1-5, the surgical fastener 2 includes a coil body 4 and a separately manufactured head 6 that is attached to a proximal end of the coil body 8. The distal end of the coil body 10 may be configured for penetrating an implantable prosthesis, tissue, muscle, and/or bone. In one example, the distal end 10 may include a sharp distal tip, although the distal end may employ any suitable configuration as should be appreciated by one of skill.

The coil body 4 includes a plurality of coil windings 12. As illustrated, the coil windings 12 may be arranged in a helical or spiral configuration suitable for driving the fastener into and through prosthetic material, tissue, muscle and/or bone. Thus, the coil body may be cylindrical in shape with a circular cross-section, though other cross-sectional shapes such as triangular, rectangular, or any other appropriate shape also are contemplated. The coil body 4 may include any number of coil windings 12 with any desired spacing or pitch between the adjacent coil windings and any diameter, or other appropriate transverse dimension, including outer, inner and pitch diameters, suitable for a particular application as should be appreciated by one of skill. In one embodiment, the coil body 4 may include coil windings 12 having the same transverse dimension. However, if desired, one or more of the coil windings may have different transverse dimensions relative to each other. For example, the coil body may employ coil windings 12 that decrease in transverse dimension from the proximal end toward the distal end to form a coil body 4 with a conical or tapered shape as illustrated in FIGS. 1 and 2.

It should be understood that the head may have any suitable configuration desired for a particular application. For example, in one example, the head includes, but is not limited to, a generally flat distal face from which extends the coil body. The opposite or proximal face of the head may have one or more generally flat, round, angled or beveled surfaces, or combinations thereof, as should be apparent to one of skill.

In embodiments of the invention, the head includes at least two radial wings 14 extending in an outward radial direction from the head body 16. Each wing 14 includes one or more external threads to engage with a corresponding internal thread of a delivery device. The wings 14 extend radially outwards by an appropriate distance such that they have a maximum outer transverse dimension that is larger than a maximum outer transverse dimension of the coil body. In such an arrangement, the coil windings may not interact with the internal thread of the delivery device when the external threads of the wings 14 engage the internal thread. It is to be appreciated that the wings may employ any suitable configuration that should be apparent to one of skill in the art.

To facilitate rotation of the fastener, the head 6 includes one or more drive features that may be engaged by a drive member of a delivery device to rotate the head, and thereby rotate the fastener including the coil body 4. Each wing 14 is spaced from an adjacent wing 14 by an opening 18, such as a slot, therebetween that is adapted to receive a drive member for driving the surgical fastener, see FIG. 8. The coil body 4 may have a transverse dimension that is smaller than the head body 16 so that the coils do not interfere with the drive member received within the slots. It is to be appreciated that the head 6 may include any number of drive features having any suitable configuration as should be apparent to one of skill.

Again referring to FIGS. 1-5, in one illustrative example, the coil body 4 may be attached to the center of the head 6. This arrangement may permit maximum use of the entire length of the coil body 4 by permitting over-compression or over-driving of the surgical fastener head 6 into an underlying prosthetic material and/or tissue while reducing, if not eliminating, the potential for ripping or tearing of the material and/or tissue as compared to a fastener in which the coil body terminates closer to the outer periphery of the head 6. Without wishing to be bound by theory, this may be due to the reduced moment arm, and thus reduced torque, present at the center of the head as it is rotated. However, it is to be appreciated that it is not required for the coil body 4 to be attached directly at the center of the head 6 in each example of the surgical fastener and that other attachment configurations may be used as should be apparent to one of skill. For example, the coil body 4 might be attached to the head 6 at any desirable point between the center of the head and the outer perimeter of the head.

It is desirable to reduce, if not eliminate, the potential for pinching a prosthetic material and/or tissue between the coil body and the head. As shown in FIGS. 1-5, the proximal end of the coil body 8 is attached to the head 6 with a proximal-most coil winding 20 being spaced from the head to form a gap therebetween. This arrangement may be implemented in place of or together with the center attachment described above. The fastener may employ a gap of approximately 0.002 inches to approximately 0.005 inches (0.05 mm to 0.13 mm), although a gap of any suitable size may be employed as should be apparent to one of skill.

The head may be attached to the coil body by molding, or otherwise attaching, a non-winding feature of the coil body 8 and head 6 together. For example, the head may be molded to a prefabricated coil body using an insert molding or over-molding process as should be apparent to one of skill.

In an illustrative embodiment of the invention shown in FIGS. 10-14, the coil body 4 includes a non-winding feature such as a central post 22 at the proximal end 8 that is attached to the head, such as by molding. A corresponding cavity 24 is formed in the head 6 during molding to accommodate and retain the central post 22 is illustrated in FIG. 10. The central post 22 includes an axial portion and a radial portion that extends from a proximal end of the axial portion. The radial portion may be oriented so as to extend in a direction toward one of the wings to maximize its length for enhanced holding between the head and the coil body, though other directions are also possible. Without wishing to be bound by theory, employing a longer radial portion may increase the amount of torque that may be applied to the coil body through the head due to the increased area the torque is applied to and the increased attachment strength..

The surgical fastener may be configured to reduce, if not prevent, post-operative back out of the fastener. In some illustrative embodiments, the head 6 may include one or more features 26 that permit tissue ingrowth into or through the head to help anchor the head in position. In one embodiment, the head 6 may include features 26 for tissue ingrowth such as one or more openings, holes, recesses, cavities, slots and the like that are configured to permit ingrowth into and/or through the head 6 as should be apparent to one of skill in the art. However, it is to be appreciated that such ingrowth features are not required for each embodiment of the surgical fastener. Additionally, in some embodiments features 26 may be used as driving features that engage with suitably shaped and arranged guide rods to drive the surgical fastener.

In one embodiment, the surgical fastener may include a coil body having a length of approximately 3 mm (0.118 inches) to approximately 5.5 mm (0.217 inches) extending from the distal face of the head. The coil body may include approximately 2.5 turns to approximately 6 turns of coils having a maximum outer transverse dimension of approximately 2.5 mm (0.100 inches) to approximately 4.9 mm (0.193 inches), for delivery through a 5 mm cannula, with a pitch of approximately 0.7 mm (0.03 inches) to approximately 1.1 mm (0.045 inches). The head may include wings with an external thread that corresponds to the internal thread of a delivery device. The head body may have a maximum outer transverse dimension of approximately 4 mm (0.157 inches) to approximately 4.9 mm (0.193inches) at the slots and a thickness of approximately 0.97 mm (0.030 inches) to approximately 1.02 mm (0.04 inches). The head may have a thickness of approximately 0.97 mm (0.030 inches) to approximately 1.02 mm (0.04 inches). The radial portion of the center post may have a length of approximately 3.05 mm (0.12 inches) to approximately 3.30 mm (0.13 inches) and may be located about midway between the proximal and distal faces of the head. Of course, the surgical fastener may employ a coil body and head having any suitable sizes and configurations for a desired application as should be apparent to one of skill in the art.

The surgical fastener may be made from one or more biocompatible materials that are suitable for a particular surgical application and is sterilized or sterilizable. The components of the fastener may be made from a non-absorbable material, an absorbable material or a combination of absorbable and non-absorbable materials. The components may be made from, and/or coated with, materials and/or include features that may resist tissue ingrowth and/or adhesions, permit tissue ingrowth and/or adhesions, or a combination thereof. The components may be made from metal, plastic and/or any other suitable materials as should be apparent to one of skill in the art.

In one embodiment, the head may be made from a plastic polymer including, but not limited to, polyether ether ketone (PEEK) or acetal, and the coil body may be made from a metal including, but not limited to, stainless steel, nitinol, or titanium. If desired, the head alone or the head and the coil body may be made from an absorbable material metal and/or polymer. Other materials for both the head and coil body are possible.

In one exemplary embodiment, the surgical fastener may has an overall length of approximately 4.5 mm (0.177 inches) with a coil length extending from the head of approximately 3.5 mm (0.138 inches). The head has a thickness of approximately 1 mm and an outer transverse dimension of approximately 4 mm (0.157 inches) to 4.25 mm (0.167 inches) at the slots. The coil body has an outer transverse dimension of approximately 2.7 mm (0.105 inches) and is formed of 0.46 mm (0.018 inch) metal wire to have a constant pitch of approximately 0.91 mm (0.036 inches) to 1.07 mm (0.042 inches).

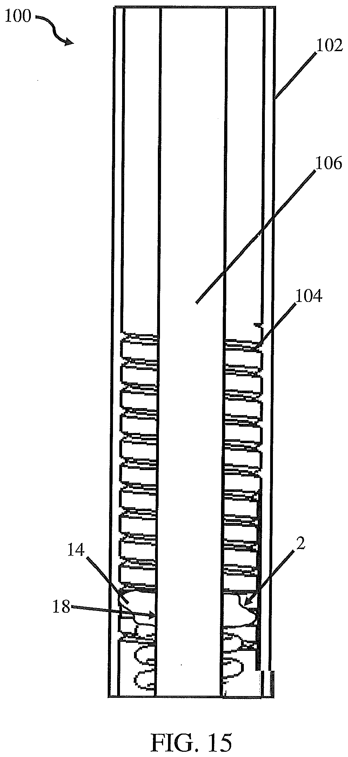

The surgical fastener may be delivered to a surgical site using a delivery device that imparts rotation to the fastener and drives the fastener into prosthetic material, tissue, muscle and/or bone. As shown in FIG. 15, the delivery device 100 may include an outer tube or cannula 102 for supporting and/or guiding one or more fasteners 2 within the cannula. The outer tube 102 may include an internal thread 104 that corresponds to and engages the external thread of the head located on wings 14. One or more drive members 106 may extend along the length of the outer tube 102 and mate with the slots 18 of each fastener. The drive members 106 may be rotated about the longitudinal axis of the outer tube to rotate the head of the fastener and thereby rotate each fastener 2 within the outer tube 102. Rotation of the head 6 relative to the internal thread 104 of the outer tube 102 in turn provides a reactive thrust to the fastener causing the fastener to be driven in a distal direction along the length of the drive members 106, out of the outer tube 102 and into the prosthetic material, bone, muscle and/or tissue. However, it is to be appreciated that the surgical fastener may be delivered using other suitable arrangements and delivery devices as should be apparent to one of skill in the art. For example, the surgical fasteners could be used in delivery devices such as a laparoscopic device, an endoscopic device, a borescopic device, a catheter, a surgical instrument for use in "open" procedures, or any other appropriate surgical instrument.

It should be understood that the foregoing description of various aspects of at least one embodiment of the invention are intended merely to be illustrative thereof and that other embodiments, modifications, and equivalents of the invention are within the scope of the invention recited in the claims appended hereto. Accordingly, the foregoing description and drawings are by way of example only.

## Claims

1. A surgical fastener comprising:
a head (6) including a head body (16) and at least two radial wings (14) extending in an outward radial direction from the head body (16), each wing (14) including at least one external thread to engage with a corresponding internal thread of a delivery device, each wing (14) being spaced from an adjacent wing (14) by an opening (18) therebetween that is adapted to receive a drive member for driving the surgical fastener; and
a coil body (4) that is separate from, and is attached to, the head (6), the coil body (4) including a plurality of coil windings (12), the coil body (4) having a proximal end and a distal end, the proximal end being attached to a center portion of the head (6),
**characterised in that** the head (6) and the coil body (4) are configured so that a gap is formed between the proximal-most coil winding (20) of the coil body (4) and the head (6);
the coil body (4) includes a central post (22) at the proximal end that is attached to the center portion of the head (6); and
the central post (22) includes an axial portion and a radial portion that extends from a proximal end of the axial portion;
wherein the radial portion is configured for enhanced holding between the head (6) and the coil body (4).

2. The surgical fastener according to claim 1, wherein a maximum outer transverse dimension of the coil body (4) is smaller than a maximum outer transverse dimension of the wings (14) so that the coil body (4) does not interact with the internal thread of the delivery device when the external threads of the wings (14) engage the internal thread.

3. The surgical fastener according to any of claims 1-2, wherein the head body (16) is constructed and arranged to support tissue ingrowth.

4. The surgical fastener according to any of claims 1-3, wherein the head body (16) includes one or more openings (18) adapted to receive tissue ingrowth.

5. The surgical fastener according to any of claims 1-4, wherein the head (6) and the coil body (4) are fabricated from different materials.

6. The surgical fastener according to claim 5, wherein the head (6) comprises a plastic or metal material.

7. The surgical fastener according to any of claims 1-6, wherein a maximum outer transverse dimension of the coil body (4) is smaller than a maximum outer transverse dimension of the head (6) so that the coil windings (12) do not interact with the internal thread of the delivery device when the at least one external thread engages the internal thread.

8. The surgical fastener according to any of claims 1-7, wherein the gap is between 0.05 mm and 0.13 mm inclusively.

9. The surgical fastener according to any of claims 1-8, wherein the coil body (4) extends between 3 mm to 5.5 mm inclusively from a distal face of the head (6).

10. The surgical fastener according to any of claims 1-9, wherein a maximum outer transverse dimension of the coil body (4) is between 2.5 mm to 4.9 mm inclusively.

11. The surgical fastener according any of claims 1-10, wherein a maximum outer transverse dimension of the head (6) is between 4 mm to 4.9 mm inclusively.

## Patentansprüche

1. Chirurgisches Befestigungselement, umfassend:
einen Kopf (6), beinhaltend einen Kopfkörper (16) und mindestens zwei radiale Flügel (14), die sich in einer nach außen ausgerichteten radialen Richtung von dem Kopfkörper (16) erstrecken, wobei jeder Flügel (14) mindestens ein Außengewinde beinhaltet, um ein entsprechendes Innengewinde einer Abgabevorrichtung in Eingriff zu nehmen, wobei jeder Flügel (14) von einem benachbarten Flügel (14) durch eine Öffnung (18) dazwischen beabstandet ist, die dazu eingerichtet ist, ein Antriebselement zum Antreiben des chirurgischen Befestigungselements aufzunehmen; und
einen Spiralenkörper (4), der separat zu dem Kopf (6) ist und an diesem angebracht ist, wobei der Spiralenkörper (4) mehrere Spiralenwindungen (12) beinhaltet, wobei der Spiralenkörper (4) ein proximales Ende und ein distales Ende aufweist, wobei das proximale Ende an einem mittleren Abschnitt des Kopfs (6) angebracht ist,
**dadurch gekennzeichnet, dass** der Kopf (6) und der Spiralenkörper (4) so konfiguriert sind, dass eine Lücke zwischen der proximalsten Spiralenwindung (20) des Spiralenkörpers (4) und dem Kopf (6) gebildet wird;
der Spiralenkörper (4) eine zentrale Stange (22) am proximalen Ende beinhaltet, die am mittleren Abschnitt des Kopfs (6) angebracht ist; und
die zentrale Stange (22) einen axialen Abschnitt und einen radialen Abschnitt, der sich von einem proximalen Ende des axialen Abschnitts erstreckt, beinhaltet;
wobei der radiale Abschnitt für ein verstärktes Halten zwischen dem Kopf (6) und dem Spiralenkörper (4) konfiguriert ist.

2. Chirurgisches Befestigungselement nach Anspruch 1, wobei ein maximales äußeres Quermaß des Spiralenkörpers (4) kleiner als ein maximales äußeres Quermaß der Flügel (14) ist, so dass der Spiralenkörper (4) nicht mit dem Innengewinde der Abgabevorrichtung zusammenwirkt, wenn die Außengewinde der Flügel (14) das Innengewinde in Eingriff nehmen.

3. Chirurgisches Befestigungselement nach einem der Ansprüche 1-2, wobei der Kopfkörper (16) dazu konstruiert und angeordnet ist, ein Gewebeeinwachsen zu unterstützen.

4. Chirurgisches Befestigungselement nach einem der Ansprüche 1-3, wobei der Kopfkörper (16) eine oder mehrere Öffnungen (18) beinhaltet, die dazu eingerichtet sind, ein Gewebeeinwachsen aufzunehmen.

5. Chirurgisches Befestigungselement nach einem der Ansprüche 1-4, wobei der Kopf (6) und der Spiralenkörper (4) aus unterschiedlichen Materialien gefertigt sind.

6. Chirurgisches Befestigungselement nach Anspruch 5, wobei der Kopf (6) ein Kunststoff- oder Metallmaterial umfasst.

7. Chirurgisches Befestigungselement nach einem der Ansprüche 1-6, wobei ein maximales äußeres Quermaß des Spiralenkörpers (4) kleiner als ein maximales äußeres Quermaß des Kopfs (6) ist, so dass die Spiralenwindungen (12) nicht mit dem Innengewinde der Abgabevorrichtung zusammenwirken, wenn das mindestens eine Außengewinde das Innengewinde in Eingriff nimmt.

8. Chirurgisches Befestigungselement nach einem der Ansprüche 1-7, wobei die Lücke zwischen 0,05 mm und 0,13 mm einschließlich liegt.

9. Chirurgisches Befestigungselement nach einem der Ansprüche 1-8, wobei der Spiralenkörper (4) sich zwischen 3 mm und 5,5 mm einschließlich von einer distalen Fläche des Kopfs (6) erstreckt.

10. Chirurgisches Befestigungselement nach einem der Ansprüche 1-9, wobei ein maximales äußeres Quermaß des Spiralenkörpers (4) zwischen 2,5 mm und 4,9 mm einschließlich liegt.

11. Chirurgisches Befestigungselement nach einem der Ansprüche 1-10, wobei ein maximales äußeres Quermaß des Kopfs (6) zwischen 4 mm und 4,9 mm einschließlich liegt.

## Revendications

1. Dispositif de fixation chirurgical comprenant :
une tête (6) comportant un corps de tête (16) et au moins deux ailes radiales (14) qui s'étendent dans une direction radiale vers l'extérieur depuis le corps de tête (16), chaque aile (14) comportant au moins un filetage extérieur pour venir en prise avec un filetage intérieur correspondant d'un dispositif de délivrance, chaque aile (14) étant espacée d'une aile adjacente (14) par une ouverture (18) entre elles qui est adaptée pour recevoir un élément d'entraînement pour entraîner le dispositif de fixation chirurgical ; et
un corps spiralé (4) qui est séparé de, et qui est fixé à, la tête (6), le corps spiralé (4) comportant une pluralité d'enroulements de spirale (12), le corps spiralé (4) ayant une extrémité proximale et une extrémité distale, l'extrémité proximale étant fixée à une partie centrale de la tête (6),
**caractérisée en ce que** la tête (6) et le corps spiralé (4) sont configurés de façon à former un espace entre l'enroulement de spirale le plus proximal (20) du corps spiralé (4) et la tête (6) ;
le corps spiralé (4) comporte à son extrémité proximale une tige centrale (22) qui est fixée à la partie centrale de la tête (6) ; et
la tige centrale (22) comporte une partie axiale et une partie radiale qui s'étend depuis une extrémité proximale de la partie axiale ;
dans lequel la partie radiale est configurée pour un maintien renforcé entre la tête (6) et le corps spiralé (4) .

2. Dispositif de fixation chirurgical selon la revendication 1, dans lequel une dimension transversale extérieure maximale du corps spiralé (4) est plus petite qu'une dimension transversale extérieure maximale des ailes (14), de sorte que le corps spiralé (4) n'interagit pas avec le filetage intérieur du dispositif de délivrance lorsque les filetages extérieurs des ailes (14) sont en prise avec le filetage intérieur.

3. Dispositif de fixation chirurgical selon l'une quelconque des revendications 1 à 2, dans lequel le corps de tête (16) est construit et disposé pour favoriser l'interposition tissulaire.

4. Dispositif de fixation chirurgical selon l'une quelconque des revendications 1 à 3, dans lequel le corps de tête (16) comporte une ou plusieurs ouvertures (18) adaptées pour recevoir l'interposition tissulaire.

5. Dispositif de fixation chirurgical selon l'une quelconque des revendications 1 à 4, dans lequel la tête (6) et le corps spiralé (4) sont fabriqués dans des matériaux différents.

6. Dispositif de fixation chirurgical selon la revendication 5, dans lequel la tête (6) comprend un matériau plastique ou métallique.

7. Dispositif de fixation chirurgical selon l'une quelconque des revendications 1 à 6, dans lequel une dimension transversale extérieure maximale du corps spiralé (4) est plus petite qu'une dimension transversale extérieure maximale de la tête (6), de sorte que les enroulements de spirale (12) n'interagissent pas avec le filetage intérieur du dispositif de délivrance lorsque ledit au moins un filetage extérieur est en prise avec le filetage intérieur.

8. Dispositif de fixation chirurgical selon l'une quelconque des revendications 1 à 7, dans lequel l'espace mesure entre 0,05 mm et 0,13 mm inclusivement.

9. Dispositif de fixation chirurgical selon l'une quelconque des revendications 1 à 8, dans lequel le corps spiralé (4) s'étend entre 3 mm à 5,5 mm inclusivement depuis une face distale de la tête (6).

10. Dispositif de fixation chirurgical selon l'une quelconque des revendications 1 à 9, dans lequel une dimension transversale extérieure maximale du corps spiralé (4) mesure entre 2,5 mm à 4,9 mm inclusivement.

11. Dispositif de fixation chirurgical selon l'une quelconque des revendications 1 à 10, dans lequel une dimension transversale extérieure maximale de la tête (6) mesure entre 4 mm à 4,9 mm inclusivement.
